# EUROPEAN PATENT APPLICATION

(11) **EP 4 094 753 A1**
(43) Date of publication of application: **30.11.2022**
(21) Application number: 21176113.5
(22) Date of filing: 27.05.2021
(51) Int. Cl.: A61K 9/48, A61K 9/16, A61K 31/785, A61P 1/00

(54) **PERFORATED CHOLESTYRAMINE CAPSULE**

(71) Applicant: Stangl, Manfred, 82054 Sauerlach (DE); Karcz, Konrad W., 81245 München (DE)
(72) Inventor: Stangl, Manfred, 82054 Sauerlach (DE); Karcz, Konrad W., 81245 München (DE)
(74) Representative: Hertin und Partner Rechts- und Patentanwälte PartG mbB

(57) **Abstract**

The invention relates to a pharmaceutical composition comprising a perforated capsule and an endotoxin-binding granule, characterized in that the granule is comprised in the capsule and the size of the granules prevents the granule from escaping from the pores of the capsule. The invention relates preferably to such composition wherein the endotoxin-binding granule is cholestyramine granule. In another aspect the invention relates to a pharmaceutical composition for use in the treatment of a microbiome-associated disease.

## Description

The present invention is in the field of compositions, formulations for pharmaceutical agents.

The invention relates to a pharmaceutical composition comprising a perforated capsule and endotoxin-binding granules, characterized in that the granules are comprised in the capsule and the size of the granule prevents the granules from escaping from the pores of the capsule. The invention relates preferably to such a composition wherein the endotoxin-binding granules are cholestyramine granules. In another aspect the invention relates to a pharmaceutical composition of the invention for use in the treatment of a microbiome-associated disease.

### BACKGROUND OF THE INVENTION

The human microbiome impacts human physiology and contributes to the enhancement and impairment of metabolic and immune functions. Changes in the immune environment may be directly linked to a dysbiotic flora of the gut. Life-threatening health conditions ranging from cancer, cardiovascular disease, bowel inflammatory disease and difficult-to-treat bacterial infections due to antibiotic resistance are associated with dysbiosis.

One aspect of microbiome-associated diseases is cancer. The microbiome carries out biochemical activities influencing carcinogenesis, tumor development, and response to immune therapy (T. Elizabeth and J. Nathalie, "Introduction to the human gut microbiota," Biochemical Journal, vol. 474, no. 11, pp. 1823-1836, 2017.). According to a well-studied model on factors that may contribute to dysbiosis in the gut, continuous intra-abdominal infections, antimicrobial drugs, or both may lead to an increased risk of colorectal cancer. In addition, H. pylori contributes to the risk of gastric cancer in humans. Recent findings on the interplay of the human microbiome and cancer point out Fusobacterium and Clostridium being overrepresented in individuals having gastric cancer (J. L. Round and S. K. Mazmanian, "The gut microbiota shapes intestinal immune responses during health and disease," Nature Reviews Immunology, vol. 9, no. 5, pp. 313-323, 2009.).

Another aspect of microbiome-associated diseases is inflammatory bowel disease. The accumulation of disease-causing microbiome induces or triggers an abnormal immune response against body tissues. This contributes to autoimmune diseases, bowel inflammatory disease, and other life-threatening conditions (K. Ipci, N. Altintoprak, N. B. Muluk, M. Senturk, and C. Cingi, "The possible mechanisms of the human microbiome in allergic diseases," European Archives of Oto-Rhino-Laryngology, vol. 274, no. 2, pp. 617-626, 2016.). An alteration in the host-microbiome affects this interaction leading to impairment in immune response which may result in inflammatory disorder (D. Rojo, C. Méndez-García, B. A. Raczkowska et al., "Exploring the human microbiome from multiple perspectives: factors altering its composition and function," FEMS Microbiology Reviews, vol. 41, no. 4, pp. 453-478, 2017.). Sunil et al. (T. Sunil, I. Jacques, W. Emily et al., "The host microbiome regulates and maintains human health: a primer and perspective for non-microbiologists," Cancer Research, vol. 77, no. 8, pp. 1783-1812, 2017.) describe the relationship of the gut microbiome with a compromise in the integrity of the gastrointestinal barrier in inflammatory bowel disease (IBD).

Further complex microbiome-associated diseases are cardiovascular diseases. Production of metabolites by the gut microbiota does not only affect the gut but also act systemically. For example, the production of trimethylamine N-oxide (TMAO) metabolites by certain gastrointestinal organisms may be implicated in heart disease (W. H. W. Tang, Z. Wang, D. J. Kennedy et al., "Gut microbiota-dependent TrimethylamineN-oxide (TMAO) pathway contributes to both development of renal insufficiency and mortality risk in chronic kidney disease," Circulation Research, vol. 116, no. 3, pp. 448-455, 2015.). Also, intestinal dysbiosis has been discovered to be associated with cardiovascular diseases, since a disrupted intestinal flora correlated with individuals having a higher risk of cardiovascular disease (Z. Y. Kho and S. K. Lal, "The human gut microbiome-a potential controller of wellness and disease," Frontiers in Microbiology, vol. 9, p. 1835, 2018; J. Li, F. Zhao, Y. Wang et al., "Gut microbiota dysbiosis contributes to the development of hypertension," Microbiome, vol. 5, no. 1, p. 14, 2017.). Overrepresentation of certain organisms has been found to contribute to cardiovascular diseases.

Systemic infection occurs by the continuous movement of bacteria in human hosts from the intestinal mucosa to other extraintestinal sites and can therefore be a microbiome-associated disease. The risk of a systemic disease from translocating organisms is higher in immune-compromised individuals who are hospitalized, undergoing surgery, or in trauma (L. W. Parfrey, W. A. Walters, C. L. Lauber et al., "Communities of microbial eukaryotes in the mammalian gut within the context of environmental eukaryotic diversity," Frontiers in Microbiology, vol. 5, p. 298, 2014.). Damage in the epithelium of the gut and the abuse of antibiotics disrupts the microbiota, leading to an increase in facultative anaerobes and a defect in the host immune responses. Some examples of translocating organisms associated with systemic infections include E. coli, K. pneumoniae, Enterobacter spp., P. mirabilis, Enterococcus spp., Streptococcus spp., and C. albicans (L. W. Parfrey, W. A. Walters, C. L. Lauber et al., "Communities of microbial eukaryotes in the mammalian gut within the context of environmental eukaryotic diversity," Frontiers in Microbiology, vol. 5, p. 298, 2014.). In addition, microbial translocation contributes to systemic infection by the production of uremic toxins. This discovery suggests that an alteration in the microbiota of the gut may lead to the synthesis of nitrogenous compounds which affect the integrity of the epithelial tight junction, allowing the transfer of these organisms and its toxins to other parts of the body.

Endotoxin is the major component of the outer membrane of Gram-negative intra-intestinal bacteria, contributing greatly to the structural integrity of the bacteria, and protecting the membrane from certain kinds of chemical attack. Endotoxin also increases the negative charge of the cell membrane and helps stabilize the overall membrane structure. Endotoxin induces a strong response from normal animal immune systems. Removing endotoxin or reducing the endotoxin-content of the intestinal tract is an efficient method of modifying the microbiome composition and its systemic effects and therefore is a promising approach for treating microbiome-associated diseases.

Cholestyramine is an endotoxin-binding substance. It is an insoluble quaternary amine ion exchange resin known to prevent endotoxin absorption intraluminally in the gut or from the peritoneal cavity, thereby preventing its systemic toxic effect. For example, cholestyramine binds fat-soluble and bacterial toxins and is effective in the treatment of enterotoxemia if it is given in the early stages of the disease.

Although cholestyramine works efficiently as an endotoxin-binding substance, it has not been employed as a medicament for such use. The reason appears to be the unpleasant side effect such as diarrhea, flatulence, and bloating, which are caused by cholestyramine powder being deposited in the crypts of the intestinal mucosa. Since cholestyramine cannot be broken down or metabolized, it remains in the gastrointestinal tract for prolonged periods and disrupts the normal intestinal flora and leads to the side effects, which can ultimately harm the health of the patient. All these reasons disqualify the employment of cholestyramine as a medicament for removing the endotoxin in the gastrointestinal tract in the treatment of microbiome-associated diseases.

Another concern on use of cholestyramine powder is that besides binding with bile acids and toxins, cholestyramine binds to numerous drugs such as digoxin (Cady, W.J., Rehder, T.L., Campbell, J., 1979. Use of cholestyramine in the treatment of digitoxin toxicity (Abstract). Am. J. Hosp. Pharm. 36, 92-94.), ibuprofen (El-Sayad, Y.M., al-Meshai, M.A., al-Angary, A.A., et al., 1994.The effect of colestipol and cholestyramine on the systemic clearance of intravenous ibuprofen in rabbits. J. Pharm. Pharmacol. 46, 73-75.), phenylbutazone and phenobarbitone. Cholestyramine also binds to the fat-soluble vitamins A, D, E and K. Furthermore, in human, large doses can cause severe constipation (CRC Desk Reference of Clinical Pharmacology, 1998. CRC Press.). The deposition of cholestyramine in crypt of the intestinal mucosa exacerbates the interaction of cholestyramine and the numerous drugs.

Despite the remarkable progress in understanding and treating microbiome-associated diseases, further alternative, improved, practical and safe approaches for treatment of this disease complex are needed.

### SUMMARY OF THE INVENTION

In light of the prior art the technical problem underlying the invention was the provision of a pharmaceutical composition which binds to endotoxin but does not deposit or get stuck in the gastrointestinal (Gl) tract of the subject. In other words, the problem to be solved was the provision of a pharmaceutical composition that efficiently binds to and removes endotoxin from the Gl tract and is at the same time efficiently transported through and subsequently removed from the Gl tract.

The problem is solved by the features of the independent claims. Preferred embodiments of the present invention are provided by the dependent claims.

Another objective of the invention was to provide a pharmaceutical composition for use in the treatment of a microbiome-associated disease.

The invention therefore relates to a pharmaceutical composition comprising a perforated capsule and an endotoxin-binding granule, characterized in that the granule is comprised in the capsule and the size of the granule prevents the granule from escaping from the pores of the capsule.

The present invention is based on the surprising finding that a pharmaceutical composition comprising a perforated capsule containing endotoxin-binding granule can remove endotoxin from body of the subject. Advantageously, the pharmaceutical composition can bind to endotoxin and endotoxin-producing Gram-negative bacteria and subsequently be removed from the body without being deposited or getting stuck in Gl tract. This avoids or decreases unpleasant side effects and/or prolonged unwanted interaction or binding to tissue and/or drugs.

Compared to powder, the use of endotoxin-binding granules of a defined minimal size can avoid segregation from the capsule because the granules are particles with different dimensions. The granules shall have a size greater than the size of the pore in the capsule and should not be soluble in any body fluid so that they do not escape from the capsule or dissolve in the body fluid.

In one embodiment, the endotoxin-binding granule is dry granule.

In embodiments, the (preferably dry) granules have a particle size diameter ranging from 3 to 0.05 mm, preferably from 0.85 to 0.15 mm, more preferably from 0.5 to 0.15 mm. Importantly, the granules must have a diameter that is greater than the pore size of capsule and smaller than the transverse dimension of the capsule. As used herein, the term diameter is understood as the smallest distance between two opposite sites of a particle. In the context of the invention, the granules/ particle do not have to be spherical, but can have any kind of three-dimensional geometry, as long as it can be ensured that the particles cannot exit through the pores of the capsule.

In a preferred embodiment, the endotoxin-binding granule is a cholestyramine granule.

Cholestyramine is a bile acid sequestrant, which binds bile in the gastrointestinal tract to prevent its reabsorption. Cholestyramine has also been described as an endotoxin-binding material which is used to prevent endotoxin absorption intraluminally in the gut or from the peritoneal cavity, thereby preventing systemic toxic effects of endotoxin. However, cholestyramine binds to numerous drugs, such as digoxin, ibuprofen, phenylbutazone and phenobarbitone, and also interferes with the metabolism of thyroid hormone. Furthermore, it interferes with absorption of fat-soluble vitamins such as vitamin K which can lead to derangements in clotting factors. These interactions and resulting adverse effect are important disadvantages of using cholestyramine for binding the endotoxin in the gut and prevented its use for this purpose. This also holds true for other endotoxin-binding materials, which are associated with similar side effects due to disadvantageous additional interactions.

Nevertheless, the pharmaceutical composition according to the invention is a perforated capsule containing endotoxin-binding granules, such as cholestyramine granules, overcomes many of the disadvantages. The pharmaceutical composition as described herein can remove the endotoxin efficiently without leaving the endotoxin-binding material, such as a cholestyramine powder, in the body due to the construct of the capsule comprising the granules but preventing their release. Further, the pharmaceutical composition as used herein avoids any toxic effect resulting from the prolonged interaction of remaining cholestyramine powder in the subject.

In another embodiment, the capsule comprises or consists of an indigestible shell material, preferably selected from the group consisting of thermoplastic polymers, metal, and combinations thereof, more preferably a silica-polymer.

It is advantageous to use an indigestible capsule material so that the capsule would not dissolve and thus cholestyramine is kept in the capsule and is subsequently excreted from the body with the capsule. A prolonged undesirable interaction with drugs, vitamins or other elements or tissue is efficiently prevented since the capsule is efficiently transported through the Gl tract without getting stuck in the lumen.

In a preferred embodiment, the indigestible material for capsule can be polycarbonates, more preferably aromatic polycarbonates. In another preferred embodiment, the indigestible material of the capsule can be silica polymer. Moreover, preferably the endotoxin-binding granules, such as preferably cholestyramine granules, are resistant to hydrolysis in stomach and intestine. Accordingly, the granules are not dissolved or disintegrated during the Gl passage and remain in the capsule, which is ultimately being excreted. Therefore, the invention prevents absorption or retention of endotoxin-binding material in the body.

In one embodiment, the capsule has an elongated shape. The elongated shape facilitates the maximal contact between the capsule and the granules in the capsule with the liquid containing the endotoxin and endotoxin-producing bacteria. Furthermore, elongated shapes of capsules have been proven advantageous in other applications where capsules are orally administered to a subject, because such capsules are easy to swallow. Furthermore, elongated capsules are efficiently transported through the Gl tract and excreted, as is known from capsule endoscopy used for visualization of the gastrointestinal (Gl) tract.

In a preferred embodiment, the capsule has a longitudinal dimension of 5 mm - 15 mm in length and a transverse dimension of 1 mm to 8 mm.

Longitudinal capsules of this size are small enough for oral administration (swallowing) while still being big enough to prevent the capsules from getting stuck in the Gl tract, for example in the crypts of the intestine. Accordingly, capsules of this size range are very likely to efficiently pass the Gl tract and prevent cholestyramine from remaining in the Gl tract, while still being user/patient friendly allowing easy/convenient swallowing.

In another preferred embodiment, the pore size of the capsule allows liquid, such as liquid contained in the lumen of the Gl tract, to flow there through.

Preferably, the capsule has a pore size that is suitable to ensure that the liquid or fluid in Gl tract can flow through the pore of the capsule and get in contact with the contained granules. Accordingly, the endotoxin-binding granules get in contact with endotoxin, bacteria comprising endotoxin and any substance which is supposed to be removed from the Gl tract and which are dissolved in the liquid present in the lumen of the Gl tract. At the same time, the pores must be sufficiently small to prevent the endotoxin binding granules from escaping from the capsule.

In embodiments, a suitable pore size can be determined by taking into account the surface tension of the liquid or fluid flowing through the pores, i.e. the fluids present in the lumen of the Gl tract. A suitable pore size can be determined by porometry, which is a characterization technique based on the displacement of a wetting liquid from the sample pores bay applying a gas at increasing pressure. The determining of pore size can be based on the following scheme: the sample under evaluation is wetted with a liquid to fill at least all through pores. Increasing gas (air or nitrogen) pressure (P) is applied to one side of the sample. The liquid will be expelled from each through pore according to its size (diameter, d) at a defining pressure according to the Washburn equation P*D=4*y*cosθ, wherein D is the pore size diameter, P is the pressure measured, γ is the surface tension of the wetting liquid and θ is the contact angle of the wetting liquid with the sample. Therefore, the skilled person is able to test and determine the range of pore size able to let the liquid or fluid with different viscosities to flow through the pore and get contact with cholestyramine.

In a preferred embodiment, the pore size of the capsule is in the range of 0.1 - 1.5 mm.

The pore size should be in a range facilitating the flow through of liquid or fluid in Gl as well as not larger than the smallest size of granules to avoid escape of granules from the capsule.

In one embodiment, the granules are spherical having a diameter in the range of 0.15 - 2 mm.

In embodiments, the spherical form of the granules is advantageous since production of such granules requires very low airflow compared to top spray granulation, and is therefore more suitable for post processing coating which makes coating endotoxin-binding substance on the surface of granules easier.

In some embodiment, the granules have uniform diameter ranging from 0.15 - 2 mm. In some embodiment, the diameter of granules can be different ranging from 0.15 - 2 mm.

In some embodiment, the smallest size of granules and greatest pore size can be, >0.5 mm and <0.1 mm, >0.6 and 0.1-0.6 mm, >0.7 mm and 0.1-0.7 mm, >0.8 mm and 0.1-0.8 mm, >0.9 mm and 0.1-0.9, >1.0 mm and 0.1-1.0 mm, >1.1 mm and 0.1-1.1 mm, >1.2 mm and 0.1-1.2 mm, >1.3 mm and 0.1-1.3 mm, >1.4 and 0.1-1.4 mm. > 1.5 and 0.1-1.5 mm.

In one embodiment, the capsule has a spherical form which is easy to swallow, fast to slip in Gl tract and the contact area between multiple capsules is minimized.

In another embodiment, the endotoxin-binding granules are not soluble, in particular not upon passage through the digestive tract. In embodiments, the endotoxin-binding granules do not dissolve or disintegrate upon passage through the digestive tract.

Cholestyramine granules are not soluble when passing through the digestive tract. This avoids cholestyramine granules escaping the capsule and getting stuck in the Gl tract.

The invention is based on the surprising finding that a pharmaceutical composition comprising a perforated capsule and endotoxin-binding granules binds efficiently endotoxin without inducing relevant side effects resulting from the direct and/or prolonged contact between endotoxin-binding material and the digestive tract. Furthermore, the risk of cholestyramine remaining in the Gl tract for a long time is strongly reduced, since it cannot exit the capsule and the capsule is transported through the Gl tract and excreted, usually within several hours until two weeks depending on each person's digestive system.

The invention therefore relates to a pharmaceutical composition as described herein for use in the treatment of a microbiome-associated disease.

Preferably, in the context of the pharmaceutical composition for use in the treatment of a microbiome-associated disease as described herein the one or more capsules are administered orally and comprise a therapeutically effective amounts of endotoxin-binding granules.

In a preferred embodiment, endotoxin and/or bacteria comprising endotoxin present in the digestive tract of a patient bind to the endotoxin-binding granules in the capsules and are excreted with the capsules. Not only the amounts of the endotoxin but also the endotoxin-containing gut microbiome in the digestive tract can be therefore largely reduced. Hence, administration of this pharmaceutical composition becomes an efficient method for treating microbiome-associated diseases.

In embodiments, a daily orally administered dose ranges from 2 g to 24 g of endotoxin-binding granules. This dose can be administered in one or several capsules containing the endotoxin binding granules. The dose may also be defined by the total surface of the endotoxin-biding granules, and by the endotoxin-binding capacity of the granules, which can be defined by the amount endotoxin bound per surface area.

In embodiments, the microbiome-associated disease is selected from the group consisting of atherosclerosis, thrombosis, cardiovascular disease, cancer, diabetes mellitus, immune-mediated inflammatory bowel disease, kwashiorkor, liver disease, metabolic syndrome, obesity, neurodevelopmental disorders, psychiatric disorders, and neurodegenerative disorders.

Embodiments and features that are disclosed in one aspect of the invention also read on the other aspects of the invention. For example, features described with respect to a pharmaceutical composition comprising a perforated capsule and an endotoxin-binding granule read on the claimed pharmaceutical composition for use in the treatment of a microbiome-associated disease and vice versa.

The various aspects of the invention are all based on the unifying concept of a pharmaceutical composition comprising a perforated capsule and an endotoxin-binding granule and the use thereof for treating a microbiome-associated disease, since it was surprisingly found that such pharmaceutical compositions are fully functional and advantageous for such applications.

### DETAILED DESCRIPTION OF THE INVENTION

The present invention relates to a pharmaceutical composition comprising a perforated capsule and an endotoxin-binding granule, characterized in that the granule is comprised in the capsule and the size of the granules prevents the granule from escaping from the pores of the capsule.

As used herein the term "endotoxin" relates to lipopolysaccharides (LPS), which are large molecules consisting of a lipid and a polysaccharide composed of O-antigen, outer core and inner core joined by a covalent bond. Endotoxins are found in outer membrane of Gram-negative bacteria.

In some embodiment, endotoxin refers to O-antigen, the outermost domain of the LPS molecule which is a repetitive glycan polymer contained within an LPS and attached to the core oligosaccharide.

In some embodiment, endotoxin refers to the core part of LPS which contains an oligosaccharide component that attaches directly to lipid A and commonly contains sugars such as heptose and keto-deoxyoctulosonate (KDO).

In some embodiment, endotoxin refers to core part of LPS which contains non-carbohydrates components, such as phosphate, amino acids and enthanolamine substituents.

In some embodiment, endotoxin can also mean only the lipid A part of LPS which is a phosphorylated gluocosamine disaccharide decorated with multiple fatty acids. Fragments of bacterial membrane containing lipid A can be released into the circulation causing fever, diarrhea and possible fatal endotoxic shock, when bacterial cells are lysed by the immune system.

In some embodiment, endotoxins include further toxins that are confined inside a microorganism and are released only when the microorganisms are broken down or die.

As used herein the term "granule" shall mean aggregations of fine particles (granules) of powders in a mass of about spherical shape. Granules comprise one or more active substances with or without excipients. However, in the context of the invention the term "granule" or "granules" are understood to refers to any kind of plurality of particles, which are preferably about homogenous or uniform.

The granule according to the invention is preferably resistant to dissolution in the digestive tract and comprises an endotoxin-binding substance, preferably an endotoxin-binding surface, wherein preferably the granules are cholestyramine granules. The dimension of granule ranges from 0.05 mm to 3 mm, such as 0.05, 0.1, 0.15, 0.2, 0.25, 0.3, 0.35, 0.4, 0.45, 0.5, 0.55, 0.6, 0.65, 0.7, 0.75, 0,8, 0.85, 0.9, 0.95, 1, 1.05, 1,1, 1.15, 1.2, 1.25, 1.3, 1.35, 1.4, 1.45, 1.5, 1.55, 1.6, 1.65, 1.70, 1.75, 1.8, 1.85, 1.9, 1.95, 2.0, 2.05, 2.1, 2.15, 2.2, 2.25, 2.3, 2.35, 2.4, 2.45, 2.5, 2.55, 2.6, 2.65, 2.7, 2.75, 2.8, 2.85, 2.9, 2.95, 3 mm, preferably 0.2 mm-2 mm, more preferably 0.25 mm-1 mm.

Granules can be used to enhance the uniformity of the active pharmaceutical ingredient in the final product, to increase the density of the blend so that it occupies less volume per unit weight for better storage and shipment, to facilitate metering or volumetric dispensing, to reduce dust during granulation process to reduce toxic exposure and process-related hazards, and to improve the appearance of the product. Consequently, the ideal characteristics of granules include spherical shape for improved flow, narrow particle size distribution for content uniformity and volumetric dispensing, sufficient fines to fill void spaces between granules for better compaction and compression characteristics, and adequate moisture and hardness to prevent breaking and dust formation during process.

In some embodiment, granule is a dry granule. As disclosed in EP0278464A1, dry granules are approximately devoid of water in which the endotoxin-binding substance is immobilized by a gum, wherein the gum can be for example hydroxypropylmethyl cellulose or methylcellulose. When the dry granules are added to an aqueous media, approximately the same number of endotoxin-binding particles continue to remain immobilized by the gum. Thus, the entire amounts of endotoxin-binding granules which contained in the perforated capsule can be swollen with water without that either the granule or the endotoxin binding substance dissolves in the liquid or body fluid. Generally, the dry granules have a particle size diameter ranging from 0.05 to 3 mm, preferably from 0.15 to 0.85 mm, more preferably from 0.15 to 0.5 mm, most importantly greater than the pore size of capsule.

In some embodiment, stable granules can be achieved by agglomeration of particles in the production of pharmaceutical dosage forms, mostly tablets and capsules. During the granulation process, small fine or coarse particles are converted into large agglomerates called granules. Generally, granulation commences after initial dry mixing of the necessary powder ingredients along with the active pharmaceutical ingredient (API), so that a uniform distribution of each ingredient throughout the powder mixture is achieved. Although granules used in the pharmaceutical industry have particle size in the range of 0.2-4.0 mm, they are primarily produced as an intermediary with a size range of 0.2-0.5 mm to be either packed as a dosage form or be mixed with other excipients before tablet compaction or capsule filling.

In one embodiment, endotoxin-binding granules can be manufactured by granulation known in the art. Endotoxin-binding substance in form of particle can be enlarged to a desired size by agglomeration and becomes endotoxin-binding granule. The granulation of endotoxin-binding substance commences after initial dry mixing of powder ingredients along with the endotoxin-binding granule, so that a uniform distribution of each ingredient throughout the powder mixture can be achieved.

Further, granulation is an exemplary of particle design and the properties of the particles acquired after granulation depend on particle size of the drug and excipients, the type, concentration, and volume of binder and/or solvents, granulation time, type of granulator, drying rate (temperature and time), etc. The primary methods by which the agglomerated granules are formed include solid bridges, sintering, chemical reaction, crystallization and deposition of colloidal particles. Besides, binding can also be accomplished through adhesive and cohesive forces by utilizing high viscous binders. The series of mechanisms by which granules are formed from the powder particles encompass wetting and nucleation, coalescence or growth, consolidation, and attrition or breakage.

In some embodiment, the endotoxin-binding substance can be coated on the granules produced in the way of above-mentioned design. The properties of the endotoxin-binding granules after granulation depend on several factors such as particle size, excipients, granulation time, dry rate. The agglomerated granules can be formed through solid bridges, sintering, chemical reaction, crystallization and deposition of colloidal particles. The coating of endotoxin-binding granules on the surface of the granules can be achieved by means of adhesive and cohesive forces by utilizing high viscous binders.

The granulation technique may be widely categorized in to two types, dry granulation and wet granulation, based on the type of method used to facilitate the agglomeration of powder particles. Dry granulation uses mechanical compression (slugs) or compaction (roller compaction) to facilitate the agglomeration of dry powder particles, while the wet granulation uses granulation liquid (binder/solvent) to facilitate the agglomeration by formation of wet mass by adhesion. Among these two techniques, wet granulation is the most widespread granulation technique used despite the fact that it involves multiple unit processes such as wet massing, drying and screening, which are complex, time consuming, and expensive requiring large space and multiple equipment.

The type of process selection requires thorough knowledge of physicochemical properties of the drug, excipients, required flow and release properties, etc. Granulation technologies like roller compaction, spray drying, supercritical fluid, low/high shear mixing, fluid bed granulation, extrusion /spheronization, etc. have been successful for many decades in the preparation of various pharmaceutical dosage forms. Pharmaceutical granulation technology continues to change, and various improved, modified, and novel techniques and technologies have been made available along the course. The aim of this review is to give the reader a glimpse of the latest techniques and technologies with regard to pharmaceutical granulation. Subsequently, this review gives a short description about each development along with its significance and limitations, which are summarized in Shanmugam 2015 (Granulation techniques and technologies: recent progresses, Biolmpacts, 2015, 5(1), 55-63).

The process of granulation is well known to the skilled person and is known from the prior art, e.g. Cantor et al., 2008 (pharmaceutical granulation processes, mechanism, and the use of binders) and Shanmugam 2015 (granulation techniques and technologies: recent progresses, Biolmpacts, 2015, 5(1), 55-63)

As used herein the term "cholestyramine" (also called Poly(trimethylammonio methylstyrolchlorid-co-divinylbenzol) or cholestyraminum) refers to an insoluble quartenary amine ion exchange resin. It is a bile acid sequestrant, which binds bile in the gastrointestinal tract to prevent its reabsorption. Cholestyramine is a strong ion exchange resin, which means it can exchange its chloride anions with anionic acids of endotoxin, such as keto-deoxyoctulosonate in the gastrointestinal tract and bind them strongly to the granules. Cholestyramine granules binds the endotoxin and/or bacteria comprising surface endotoxin by forming insoluble complexes with endotoxin in the intestine, which are subsequently excreted in the feces.

In some embodiment, cholestyramine is a synthetic, strongly basic anion exchange resin containing quaternary ammonium functional groups which are attached to a styrene-divinylbenzene copolymer, such as described in US 3308020, US 3383281, US 3499960, US 3974272.

Cholestyramine is commonly used to treat diarrhea resulting from bile acid malabsorption. It was first used for this in Crohn's disease patients who had undergone ileal resection. The terminal portion of the small bowel (ileum) is where bile acids are reabsorbed. When this section is removed, the bile acids pass into the large bowel and cause diarrhea due to stimulation of chloride/fluid secretion by the colonocytes resulting in a secretory diarrhea. Cholestyramine prevents this increase in water by making the bile acids insoluble and osmotically inactive.

Further, cholestyramine is also used in the control of other types of bile acid diarrhea. The primary, idiopathic form of bile acid diarrhea is a common cause of chronic functional diarrhea, often misdiagnosed as diarrhea-predominant irritable bowel syndrome (IBS-D), and most of these patients respond to Cholestyramine. It is beneficial in the treatment of post-cholecystectomy syndrome chronic diarrhea. Cholestyramine is also useful in treating post-vagotomy diarrhea.

Moreover, cholestyramine can be helpful in the treatment of Clostridium difficile infections, to absorb toxins A and B, and reduce the diarrhea and the other symptoms these toxins cause.

The term "endotoxin-binding granule" refers to granule comprising endotoxin-binding substance. In the context of the present invention, the interaction between endotoxin binding granule and endotoxin can eliminate the endotoxin in Gl tract or can prevent endotoxin absorption intraluminally in the gut thereby preventing its systemic toxic effect.

In embodiments, the endotoxin-binding substance is selected from but not limited to polymyxin B, poly-L-lysine or chitosan. In some embodiment, the endotoxin-binding substance can bind to any part of the endotoxin, including lipid A, or core-oligosaccharides or O-antigen. In some embodiment, the endotoxin-binding substance can be an isolated polypeptide from bacteriophages, or synthesized antibody against any part of the endotoxin, or polymer. In embodiments, the endotoxin-binding substance is selective for a specific part of the endotoxin, such as specific for lipid A, core-oligosaccharides or O-antigen.

In a preferred embodiment, the interaction between endotoxin-binding granule and endotoxin is through a covalent bond resulting in an irreversible binding. In embodiments, the interaction between endotoxin-binding granule and endotoxin is through non-covalent binding.

In some embodiment, the granule is an endotoxin binding substance.

Cholestyramine binds not only to endotoxin but also to numerous drugs such as digoxin (Cady, W.J., Rehder, T.L., Campbell, J., 1979. Use of cholestyramine in the treatment of digitoxin toxicity (Abstract). Am. J. Hosp. Pharm. 36, 92-94.), ibuprofen (El-Sayad, Y.M., al-Meshai, M.A., al-Angary, A.A., et al., 1994.The effect of colestipol and cholestyramine on the systemic clearance of intravenous ibuprofen in rabbits (Abstract). J. Pharm. Pharmacol. 46, 73-75.), phenylbutazone and phenobarbitone. It has been used to delay absorption of toxic substances from the gut in cases of toxic overdose in the dog, although activated charcoal is now considered the treatment of choice (Gfeller, R.W., Messonnier, S.P., 1997. Handbook of Small Animal Toxicology and Poisonings. Mosby). Cholestyramine also binds to the fat-soluble vitamins A, D, E and K. In human, large doses can cause severe constipation (CRC Desk Reference of Clinical Pharmacology, 1998. CRC Press.). Since cholestyramine powder usually gets stuck in the gastrointestinal tract and thus stays longer in the body. This leads to increased possibilities of interaction with other elements and prolonged these interactions. Thus, the direct oral administration of cholestyramine powder exacerbates the above-mentioned adverse reaction and harms the health of the patient.

In a preferred embodiment, the endotoxin-binding substance is cholestyramine. The disadvantages of oral administration cholestyramine powder are compromised by the perforated capsule according to the invention. The liquid or body fluid containing endotoxin or bacteria comprising endotoxin can penetrate in the perforated capsule so that the cholestyramine granules efficiently bind to endotoxin and endotoxin-comprising bacteria in the Gl tract and is excreted subsequently without getting stuck in Gl. In this embodiment, cholestyramine efficiently removes the endotoxin in Gl and reduces the above-mentioned unwanted interaction with other elements.

As used herein, the term "digestive tract" refers to the organs that food and liquids travel through when they are swallowed, digested, absorbed, and leave the body as feces. These organs include the mouth, pharynx, esophagus, stomach, small intestine, large intestine, rectum and anus. "digestive tract" and "gastrointestinal tract" in the context of present invention are interchangeable.

In some embodiments, the capsule comprises an indigestible shell material, preferably selected from the group consisting of thermoplastic polymer, silica polymer, metal and combinations thereof.

As used herein the term "capsule" refers to a range of dosage forms for enclosing medicines in a relatively stable shell, allowing them for example, to be taken orally. The capsule could be hard-shelled or soft -shelled, preferably hard-shelled.

In the context of the invention, the shell material of the capsule is indigestible. This is important because the cholestyramine granules are kept in the capsule all time from swallow until excretion. This avoids prolonged stay of cholestyramine in the Gl.

It is understood, that "indigestible" means not digestible or not easily digested in the digestive tract. In some embodiments, the capsule is constructed of substantially inert material, for example, weakened plastics, rubber, silicone, digestion-resistant starches and fibers, gelatinous material or any combination thereof. In some embodiment, the capsule comprises protein-free material. In some embodiment, the capsule is constructed of substantially a natural vegetable-derived polysaccharide, preferably, Hypromellose, cellulose, alginate, a biodegradable carbohydrate, rubber, silicone rubber, gelatinous material or a combination thereof.

In a preferred embodiment, capsule is constructed of indigestible biocompatible material, impervious to stomach acid alkaline intestinal flora, mechanically strong and safe, for example, a thermoplastic, such as polycarbonate, silica polymer, metal or any combination thereof.

In some embodiment, "indigestible" means just not completely digested in the digestive tract to the extent that the endotoxin-binding substance is still encapsulated in the capsule.

As used herein, the term "thermoplastic polymer" refers to polymers that can be melted and recast almost indefinitely. They are molten when heated and harden upon cooling. They are stable when put in the digestive tract. Thermoplastic polymers have a simple molecular structure comprising chemically independent macromolecules. Upon heating, they are softened or melted, then shaped, formed, welded, and solidified when cooled. Multiple cycles of heating and cooling can be repeated allowing reprocessing and recycling. These properties make the manufacture of the capsule, especially a perforated capsule easier. Thermoplastic polymer is selected from but not limited to group of acrylic, acrylonitrile butadiene styrene (ABC), nylon, polylactic acid, polybenzimidazole, polycarbonate (PC), polyether sulfone (PES), polyoxymethylene (POM), polyether ether ketone (PEEK), polyetherimide (PEI), polyethylene (PE), polyphenylene oxide (PPO), polyphenylene sulfide (PPS), polypropylene (PP), polystyrene, polyvinyl chloride, polyvinylidene fluoride (PVDF), Polytetrafluoroethylene (Teflon).

In some embodiment, the capsule material comprises polycarbonate. Polycarbonates (PC) are a group of thermoplastic polymers containing carbonate groups in their chemical structures. Polycarbonates used in engineering are strong, tough materials, and some grades are optically transparent. They are easily worked, molded, and thermoformed. Because of these properties, polycarbonates find many applications. Products made from polycarbonate can contain the precursor monomer bisphenol A (BPA). A balance of useful features of PC, including temperature resistance, impact resistance and optical properties, positions polycarbonates between commodity plastics and engineering plastics.

Polycarbonates are high molecular weight polymers containing carbonate moieties as linking groups for diols either in aliphatic or aromatic systems. Due to their dominant importance only aromatic polycarbonates is mostly applied. They are considered to be one of the most important engineering plastics. Polycarbonates have been known since the nineteenth century. They were synthesized by the reaction of phosgene with an aqueous solution of alkali salts of hydrochinone or resorcinol in a mixture with toluene. The first commercially available polycarbonate resin-the aliphatic crosslinkable liquid (CR 39) based on the bis-allyl carbonate of diethylene glycol- was introduced to the market by Pittsburgh Plate Glass Company (PPG) in 1941. Today the economically most important material is the aromatic polycarbonate based on 2,2-bis(4-hydroxy-phenyl)- propane (BPA). This resin was independently discovered by H. Schnell at Farbenfabrik Bayer and D. W. Fox at General Electric Company in the early 1950s. The polycarbonate based on BPA has been manufactured by Bayer AG under the trade name Makrolon since 1958. Further polycarbonate resins are commercially available from General Electric Plastics (Lexan), DOW (Calibre), Teijin (Panlite), and Mitsubishi Engineering (Jupilon).

In some embodiment, the capsule is constructed of silica-polymer composite. Silica-polymer composites are applied widely in encapsulation (Nguyen, T.-Q.; Wu, J.; Doan, V.; Schwartz, B. J.; Tolbert, S. H. Control of Energy Transfer in Oriented Conjugated Polymer-Mesoporous Silica Composites. Science 2000, 288, 652-656; Caruso, F.; Caruso, R. A.; Möhwald, H. Nanoengineering of Inorganic and Hybrid Hollow Spheres by Colloidal Templating. Science 1998, 282, 1111-1114.). Silica typically provides a physically robust, inert substrate with improved resistance to thermal, chemical, and mechanical damage when compared to bare polymer phases. Silane coupling agents provide strong covalent linkages between such disparate materials; example composites coupled via a silane intermediate include hydrophobic polymers such as poly(methyl methacrylate) and poly(styrene) or hydrophilic polymers including gelatin, and poly(-glutamic acid). In the context of the invention, the capsule constructed of silica-polymer composite is not only indigestible in the digestive tract but also chemically stable without interacting with tissue in the digestive tract or any elements in body fluid.

In a preferred embodiment, the silica-polymer composite is a silica-polyelectrolytes composite. Polyelectrolytes (both cationic and anionic) form composites with silica through a variety of mechanisms. Composite silica-poly(styrene sulfonate) (PSS) particles have been made for controlled release of potassium. By the dropwise addition of tetraethoxysilane (TEOS) to a reverse emulsion containing dispersed PSS, TEOS was found to hydrolyse and condense without the use of a catalyst to form a silica phase accounting for 70 % of the nal particle mass. Such an approach to the synthesis of a composite capsule is fundamentally simple, yet potentially highly robust, and applicable to a range of materials.

In another preferred embodiment, the silica-polymer composite is a silica-polyacrylamide. Poly(acrylamide) (PAM) is an extensively used, industrially relevant hydrophilic polymer. The functionality of poly(acrylamide) arises from its primary amide functionality. While poly(acrylamide) is a non-reactive, non-labile material, it can be readily doped during synthesis to include cationic or anionic sites. Despite the industrial appeal of the material, its amenability to the incorporation of a variety of chemical functionalities, the wide variety of materials found to allow silica deposition, the a nity of silica for amine-containing materials and the use of acrylamide-derivatives such as poly(N-isopropylacrylamide), the use of poly(acrylamide) in silica hybrids or as a template is to date quite limited. Several authors have reported using hybrid silica{poly(acrylamide) gel phases for the immobilisation or sensing of heavy metals and rare earths, but there are few examples of poly(acrylamide) microgels being used as a template or within hierarchal silica designs. However, it has been used in the templated growth for materials including titania, silver chloride nanoparticles, aluminium shells, and graphene oxide hydrogels, indicating its broad appeal as an efficient and cost-effective template for manufacturing the capsule according to the invention.

In a more preferred embodiment, the silica-polymer composite is a silica-polycarbonate.

As used herein, the term "metal" shall mean elements classified as metal according to their physical and chemical properties in the periodic table which are Li, Be, B, Na, Mg, Al, Si, K, Ca, Sc, Ti, V, Cr, Mn, Fe, Co, Ni, Cu, Zn, Ga, Ge, As, Rb, Sr, Y, Zr, Nb, Mo, Tc, Ru, Rh, Pd Ag, Cd, In Sn, Sb, Te, Cs, Ba, La, Ce, Pr, Nd, Pm, Sm, Eu, Gd, Tb, Dy, Ho, Er, Tm, Yb, Lu, Hf, Ta, W, Te, Os, Ir, Pt, Au, Hg, Ti, Pb, Bi, Po, Fr, Ra, Ac, Th, Pa, U, Np, Pu, Am, Cm, Bk, Cf, Es, Fm, Md, No, Lr, Rf, Db, Sg, BH, Hs or any alloy made with any combination thereof. In some embodiment, metal is noble metal, such as, Ru, Rh, Pd, Os, Ir, Pt, Au, Ag, Cu, Re or Hg or any alloy made with any combination thereof.

The capsule according to the invention is a perforated capsule. In this context, it is understood that "perforated" refers to a plurality of perforations. The surface where the perforations are distributed is about 10%, 20%, 30%, 40%, 50%, 60%, 70% of the whole surface of the capsule. The dimension of perforations can be unified or different. Preferably, the dimension of the perforations ranges from 0.1 to 1 mm wherein the maximal dimension of perforation should be smaller than the minimal dimension of granules to avoid the escaping of the granules from the capsule.

Preferably, the shape of capsule according to the invention could be spherical and elongated, preferably elongated. In a more preferred embodiment, the capsule has a longitudinal dimension of 5 mm, 10 mm, 12 mm, 14 mm, 16 mm, 18 mm, 20 mm, 22 mm, 24 mm, 26mm, in length, preferably, 5 mm- 15 mm and a transverse dimension of 1 mm, 2 mm, 4 mm, 6 mm, 8 mm, 10 mm, 12 mm, preferably 1 mm to 8 mm.

The pharmaceutical composition as described herein is for use in the treatment of a microbiome-associated disease. The term "microbiome" refers to the collection of microorganisms living together. Microbial communities are defined as multi-species assemblages, in which microorganisms interact with each other in a contiguous environment. The microbiome not only refers to the involved microorganisms selected from the group consist of bacteria, archaea, fungi, protists and algae but also encompass their theatre of activity including polypeptide, lipid, polysaccharide, nucleic acid, such as structural DNA/RNA, mobile genetic elements, such as viruses/phages relic DNA which results in the formation of specific ecological niches. The microbiome, which forms a dynamic and interactive micro-ecosystem prone to change in time and scale, is integrated in macro-ecosystems including eukaryotic hosts, and here crucial for their functioning and health.

In the present invention, the terms "microbiome" and "microbiota" are interchangeable.

In the context of the invention microbiome relates to gut-colonizing bacteria which stimulates the normal development of the humoral and cellular mucosal immune systems. The signals and metabolites of microorganisms can be sensed by the hematopoietic and non-hematopoietic cells of the innate immune system and translated into physiological responses. For example, the gut microbiota generates a tolerogenic response that acts on gut dendritic cells and inhibits the T-helper cell anti-inflammatory pathway.

However, some gut-colonizing microbiota induce inflammation under certain conditions. Gl tract microbiota are closely associated to infection diseases. Infection is one of the most common diseases caused by dysbiosis of the microbiota. Infectious diseases and their treatment have a profound impact on the human microbiota, which in turn determines the outcome of the infectious disease in the human host. The following example shows how the Gl microbiome plays a role in infectious disease and its treatment. The treatment of antibiotics can disturb intestinal mucosa homeostasis, thus decreasing resistance against toxin-producing *clostridium difficile* and promoting the progression of *clostridium difficile* infection (CDI). A decrease in putative butyrate-producing anaerobic bacteria and an increase in endotoxin-producing opportunistic pathogens and lactate-producing phylotypes have been detected in patients following antibiotic administration, whether or not CDI is present. Putative butyrate-producing anaerobic bacteria are significantly depleted in patient with antibiotic treatment when compared with healthy controls. These changes in Gl microbiome may increase susceptibility to *C. difficile* colonization. Therefore, the Gl microbiome plays an important role and associated with the progression of diseases, such as infectious diseases.

The Gl tract microbiome has a further impact on organs besides Gl tract. For example, Gl tract and liver have close interaction, including for example the chronic exposure of the liver to gut-derived factors including bacteria and bacterial components. The intestinal microbiota produce ethanol, ammonia, and acetaldehyde; these products may influence liver function through endotoxin release. As mentioned above, alternations in the intestinal microbiota play an important role in inducing and promoting liver damage as well as in direct liver injury resulting from different causal agents, such as viral, toxic and metabolic agents, through mechanisms such as the activation of Kupffer cells by bacterial endotoxins. The Gl tract microbiome participates in the pathogenesis of liver cirrhosis complications, such as infections, spontaneous bacterial peritonitis, hepatic encephalopathy and renal failure. Patients with liver cirrhosis have an altered bacterial composition in their gut. Thus, Gl tract microbiome contributes largely to the progression of many diseases.

In the context of the invention, the pharmaceutical composition as described herein can remove the endotoxin secreted from bacteria as well as the bacteria comprising endotoxin on its surface. This can for example help bring back the Gl microbiome balance after an antibiotic therapy or reduce damage to the liver due to the endotoxin secreted from the bacteria.

In the context of the invention, "Microbiome associated disease" shall mean any disease in which the Gl microbiome plays a role. Microbiome associated diseases are preferably selected from the group consisting of atherosclerosis, thrombosis, cardiovascular disease, cancer, diabetes mellitus, immune-mediated inflammatory bowel disease, kwashiorkor, liver disease, metabolic syndrome, obesity, neurodevelopmental disorders, psychiatric disorders, and neurodegenerative disorders. Microbiome associated disease can be caused by infection with bacteria including but not limited to Clostridium difficile, Helicobacter pylori, bacterial vaginosis.

As used herein the terms "therapeutically effective amount" and "effective amount" are interchangeable and refer to the amount or dose of the capsules of the present invention which, upon single or multiple dose administration to a patient, provides the desired or sufficient reduction of GI tract microbiome. Therapeutically effective amounts of the capsule can comprise an amount in the range of from 0.1, 0.5, 1, 2, 4, 6, 8, 10, 12, 14, 16, 18, 20, 22, 24 g of endotoxin-binding granules, preferably 2-24 g. It will be appreciated that the precise therapeutic dose will depend on the age and condition of the patient, nature of the condition to be treated and will be at the ultimate discretion of the attendant physician. Furthermore, the total surface area of the granules comprised in the capsule and the endotoxin-binding capacity of the granules (preferably measures in amount of endotoxin bound per surface area) also need to be taken into account when determining the effective dose.

As used herein the term "treatment" shall mean to obtain a desired pharmacological effect and/or physiological effect. The effect may be prophylactic in view of completely or partially preventing a disease and/or a symptom, for example, by reducing the risk of a subject having a disease or symptom or may be therapeutic in view of partially or completely curing a disease and/or adverse effect of the disease. In the context of the invention, the treatment refers to slowing, interrupting , arresting, controlling, stopping, reducing the progression of the Gl tract microbiome, but does not mean completely remove the Gl tract microbiome or cure the microbiome-associated disease. The term "treatment" and the like refers further to a therapeutic intervention that ameliorates a sign, symptom, etc., of a microbiome-associated diseases or pathological condition after it has begun to develop.

### EXAMPLES

### Example 1: synthesis of polycarbonate for use as capsule material via interfacial polycondensation

Aromatic polycarbonates can be synthesized either by interfacial polycondensation or melt condensation. For industrial manufacture most 2,2-bis(4-hydroxy-phenyl)-propane BPA polycarbonate is currently produced by interfacial polycondensation: BPA and phenolic chain regulators are dissolved in aqueous caustic, whereas methylene chloride is commonly used as immiscible second phase. The reaction is initiated by reaction of phosgene with the alkaline salts of the bisphenol. It is carried out under vigorous agitation. Carbonate oligomers are produced at the face and enter the organic phase. After addition of a tertiary amine catalyst, e.g., triethylamine, polycondensation occurs and high molecular weight polycarbonate is formed:

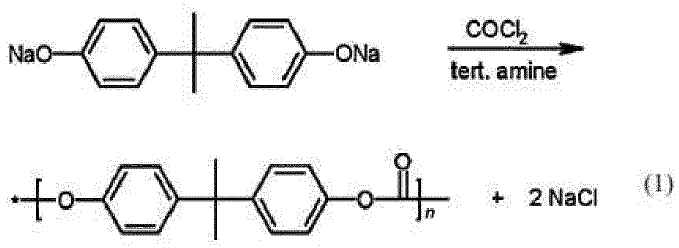

The molecular weight is controlled by the amount of chain terminator added.

### Example 2: synthesis of polycarbonate via melt condensation

Diphenyl carbonate (DPC) is used as source for the carbonate moiety if polycarbonate is produced by a melt condensation process. The polymer is obtained by transesterification with BPA:

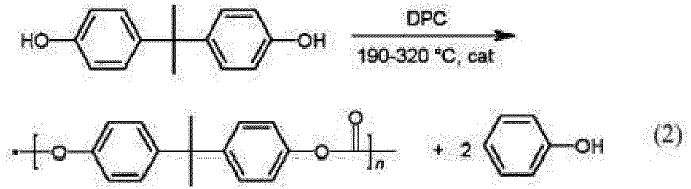

High temperatures up to 320°C and catalysts like sodium or potassium hydroxide or phosphonium compounds are necessary to achieve high molecular weight products.

### Example 3: synthesis of polymer-silica core-shell microparticle capsules

Materials:
Acrylamide (98 %), N,N'-methylenebisacrylamide (99 %), ammonium persulfate (98 %), N,N,N',N'-tetramethylethylenediamine (99 %), tetraethoxysilane (TEOS, 99 %), (3-mercaptopropyl)-trimethoxysilane (MPTMS, 95 %), cyclohexane (99%), sorbitan monooleate (70 %), poly(allylamine hydrochloride) (PAH, 4.5 105 g/mol, 99 %), poly(acrylic acid) (PAA, 4.5 105 g/mol, 99 %), sodium phosphate (99 %), iron (II) chloride tetrahydrate (99 %), iron (III) chloride (97 %), ammonium molybdate tetrahydrate (83 %) and potassium antimony tartrate (99 %) were purchased from Sigma Aldrich. Hydrochloric acid (HCI, 32 %), sodium hydroxide (NaOH, 99 %) and epichlorohydrin (99 %) were purchased from Chem-Supply (Australia). Tris(hydroxymethyl)aminomethane hydrochloride (tris HCI, 99 %) was purchased from VWR lifescience. Sodium tetrachloroaurate (III) trihydrate (99 %), sodium acetate (98 %) and I-ascorbic acid (99.7 %) were purchased from BDH Chemicals.
Acetic acid (99.7 %) and sulfuric acid (98 %) were purchased from Ajax Finechem. Trisodium citrate dihydrate (99 %), sodium chloride (NaCl, 99.5 %), di-sodium hydrogen phosphate (99 %), sodium dihydrogen phosphate (99 %), iso-propyl alcohol (99.5 %) and ethanol (96 %) were purchased from Merck Chemicals. Water was sourced from a Millipore Milli-Q water system (> 18.2 M cm). The acrylamide monomer was recrystallised from acetone before use; allother chemicals were used without further purifcation.

Synthesis:
Poly(acrylamide) microgel synthesis In a typical synthesis acrylamide (1.0 g), N,N'-methylenebisacrylamide (0.04 g) and ammonium persulfate (0.20 g) were dissolved in water (1.67 g). Sorbitan monooleate (0.10 g) was dissolved in cyclohexane (10 mL). The aqueous phase was dispersed into the organic under stirring at 1000 RPM and N,N,N',N'-tetramethylethylene-diamine (50 µL) added to the dispersion. The emulsion was left stirring for 4 hours to allow completion of the reaction.

Poly(acrylic acid)/poly(acrylamide) microgel synthesis In a typical synthesis acrylamide (1.0 g), poly(acrylic acid) (0.15 g), N,N'-methylenebisacrylamide (0.04 g) and ammonium persulfate (0.20 g) were dissolved in water (1.67 g). Sorbitan monooleate (0.10 g) was dissolved in cyclohexane (10 mL). The aqueous phase was dispersed into the organic phase under stirring at 1000 RPM and N,N,N',N'-tetramethylethylenediamine (50 µL) added to the dispersion. The emulsion was left stirring for 4 hours to allow completion of the reaction.

Silica shell deposition TEOS (1.0 g) was hydrolysed by the addition of hydrochloric acid (1 mol/L-1, 20 µL) under stirring for 5 minutes before being left to phase separate. In a typical synthesis, hydrolysed TEOS (0.1-0.5 g) was subsequently added to the poly(acrylamide) suspension as described above and gently stirred for 24 hours to allow deposition to complete. Following this reaction, the hybrid material was centrifuged, redispersed and washed in cyclohexane (3x), isopropanol (3x) and ultra-pure water (3x). Samples were stored as either aqueous dispersions under refrigeration or were freeze dried using a Labconco Freezone 2.5 and stored under an inert atmosphere prior to use.

Gold nanoparticle-decorated capsule synthesis Mercapto-functionalised silica was synthesised by the addition of MPTMS (1.0 mL) to a silica{poly(acrylamide) dispersion in ethanol, which was then left gently stirring for 24 hours to allow the reaction to come to completion. The dispersions were centrifuged, redispersed in water (3) and refrigerated prior to use. Sodium tetrachloroaurate (III) trihydrate (0.0198 g) was dissolved in water (45 mL) and brought to a rolling boil. Trisodium citrate dihydrate solution (1 w/v%, 5 mL) was added to the boiling solution and left for 5 minutes for the reaction to come to completion. The colloidal suspension was cooled on ice. The gold nanoparticle suspension (15-60 mL) was added to the mercapto-functionalised microcapsule dispersion (0.50 g dry basis) in order to produce a gold decorated microcapsule dispersion, which was refrigerated prior to use UV-vis spectrophotometry was used to determine the AuNP concentration (on a gravimetric basis) in the supernatant both prior to and following gold deposition, allowing quanti cation of the gold loading on the capsule surface.

Poly(allylamine) microgel synthesis Poly(allylamine hydrochloride)(0.50 g) and NaOH (0.05 g) were dissolved in water (1.0 g). Sorbitan monooleate (0.30 g) was dissolved in cyclohexane (10 mL). The aqueous phase was dispersed in the organic under stirring at 1000 RPM and epichlorohydrin (25 µL) added to the dispersion to cross-link the polymer, in line with previous syntheses. The emulsion was left stirring for 4 hours to allow completion of the reaction. Silica deposition was subsequently performed via the above method.

Superparamagnetic magnetite (Fe₃O₄) nanoparticle synthesis A solution (5 mL) of iron (II) chloride (0.10 mol/L) and iron (III) chloride (0.20 mol/L) was added dropwise to a NaOH solution (0.10 mol/L, 50 mL) under stirring at 1000 RPM and left to react for 30 minutes. The suspension was settled magnetically and the supernatant discarded. The iron oxide powder was centrifuged in HCI (10 mmol/L, 3x) before being dispersed in water to a final concentration of 3 g/L. For the synthesis of a magnetically responsive microgel, this suspension was used as the 'water' phase during a poly(acrylamide) synthesis, (magnetite loading 4.5 mg/g dry polymer), followed by silica deposition.

### Example 4: granulation of Cholestyramine/Methylcellulose Ether Powdered Blend

Approximately 10 lbs (4.5 kg) of a heterogeneous mixture was made by first blending three parts cholestyramine powder with one part Methocel^{®} A4M, trademark of The Dow Chemical Company, Midland, Michigan. Methocel A4M^{®} is an untreated powder of methylcellulose ether having a viscosity of 4000 centipoise. To this powder blend, water was added to make a paste having a water content of about 75 percent. The water proportionates between the cholestyramine and the Methocel^{®} powder. The cholestyramine swells but does not dissolve. The Methocel^{®} powder hydrates and dissolves forming a gelled gum which extends continuously throughout the paste to immobilize the cholestyramine particles.

This heterogeneous paste was then extruded with an RE-6 Extructor^{®} with 1/16 inch (0.16 cm) die holes, and the moist extrudate was chopped into wet pellets. The wet pellets were dried in a tray dryer and ground with a 6 inch (15 cm) Wiley knife mill using a 20 mesh (U.S. Standard) internal screen to the following particle size distribution:
A cut from 30 to 60 mesh was obtained by screening. Generally, a 35 mesh is equivalent to 500 microns and a 60 mesh is equivalent to 250 microns. The cut was blended with a flavoring agent base at 2.7 parts of the cut to 8.0 parts flavoring agent base. Then about 21 g of this blend was mixed in approximately 6 ounces (oz) water to form a smooth, good tasting orange drink with good palatability.

### Example 5: simultaneous wetting/extrusion of powdered blend of cholestyramine/methylcellulose

One part Methocel^{®} A4M powder having a viscosity of 4000 centipoise was blended with two parts cholestyramine powder at a weight ratio of 1:2 respectively. As this blend was fed to an RE-6

Extructor^{®} at a rate of 56.2 pounds per hour (Ibs/hr) (71 x 10⁻⁴ kg/s), water was fed to the Extructor^{®} at 131 Ibs/hr (165 x 10⁻⁴ kg/s) to form a heterogeneous paste mixture having a moisture content of 70 percent. This paste was extruded into moist 1/16 inch (0.16 cm) diameter strands which were chopped into 1/8 to 3/4 inch (0.3 to 1.9 cm) pellets. The pellets were dried in a fluid bed dryer and ground with a Mikropulverizer 10 ACM granules mill to the following particle size distribution.

## Claims

1. A pharmaceutical composition comprising a perforated capsule and an endotoxin-binding granule, **characterized in that** the granule is comprised in the capsule and the size of the granules prevents the granule from escaping from the pores of the capsule.

2. The pharmaceutical composition according to claim 1, wherein the endotoxin-binding granule is a cholestyramine granule.

3. The pharmaceutical composition according to any one of the preceding claims, wherein the capsule comprises an indigestible shell material, preferably selected from the group consisting of thermoplastic polymers, metal, and combinations thereof, more preferably silica-polymer.

4. The pharmaceutical composition according to any one of the preceding claims, wherein the capsule has an elongated shape.

5. The pharmaceutical composition according to the preceding claim, wherein the capsule has a longitudinal dimension of 5 mm - 15 mm in length and a transverse dimension of 1 mm to 8 mm.

6. The pharmaceutical composition according to any one of the preceding claims, wherein the pore size allows liquid to flow therethrough.

7. The pharmaceutical composition according to any one of the preceding claims, wherein the pore size is in the range of 0.1 - 1.5 mm.

8. The pharmaceutical composition according to any one of the preceding claims, wherein the granules is spherical having a diameter in the range of 0.15 - 3.0 mm.

9. The pharmaceutical composition according to any one of the preceding claims, wherein the endotoxin-binding granules are not soluble, in particular not upon passage through the digestive tract.

10. The pharmaceutical composition according to any one of the preceding claims for use in the treatment of a microbiome-associated disease.

11. The pharmaceutical composition for use in the treatment of a microbiome-associated disease according to the preceding claim, wherein one or more capsules are administered orally and comprise a therapeutically effective amounts of endotoxin-binding granules.

12. The pharmaceutical composition for use in the treatment of a microbiome-associated disease according to the preceding claim, wherein endotoxin present in the digestive tract of a patient binds to the endotoxin-binding granules in the capsules and is excreted with the capsules.

13. The pharmaceutical composition for use in the treatment of a microbiome-associated disease according to any one of claims 10-12, wherein a daily orally administered dose ranges from 2 g to 24 g of endotoxin-binding granules.

14. The pharmaceutical composition for use in the treatment of a microbiome-associated disease according to any one of claims 10-13, wherein the microbiome-associated disease is selected from the group consisting of atherosclerosis, thrombosis, cardiovascular disease, cancer, diabetes mellitus, immune-mediated inflammatory bowel disease, kwashiorkor, liver disease, metabolic syndrome, obesity, neurodevelopmental disorders, psychiatric disorders, and neurodegenerative disorders.
